(19)

Europäisches Patentamt
European Patent Office
Office européen des brevets

(11) **EP 2 924 972 A1**

(12) **EUROPEAN PATENT APPLICATION**

(43) Date of publication:
**30.09.2015 Bulletin 2015/40**

(21) Application number: **15161087.0**

(22) Date of filing: **26.03.2015**

(51) Int Cl.:
*H04N 5/217* (2011.01)          *H04N 5/32* (2006.01)
*A61B 6/00* (2006.01)          *G06T 7/00* (2006.01)
*H04N 5/367* (2011.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **27.03.2014 JP 2014066353**

(71) Applicant: **Canon Kabushiki Kaisha Tokyo 146-8501 (JP)**

(72) Inventor: **Nagai, Kentaro Tokyo, Tokyo 146-8501 (JP)**

(74) Representative: **TBK Bavariaring 4-6 80336 München (DE)**

(54) **IMAGE PROCESSING APPARATUS AND IMAGING SYSTEM**

(57)    An image processing apparatus includes: an image acquisition unit configured to acquire an original image containing a periodic pattern including a defective pixel; a compensation pattern image acquisition unit configured to acquire a compensation pattern image containing a periodic pattern same as that of the original image; a defect location identifying unit configured to identify a position of the defective pixel in the original image; and a compensation unit configured to compensate the identified defective pixel of the original image with a pixel on the compensation pattern image that is located in a position corresponding to the position of the defective pixel, to generate a compensated image in which the compensated defective pixel is compensated.

FIG.6A

**Description**

BACKGROUND OF THE INVENTION

Field of the Invention

The present invention relates to an imaging technique for capturing an image having a periodic pattern.

Description of the Related Art

The measurement methods using phases have conventionally been used as means for precisely measuring the shapes of substances. Such measurement methods using phases each have a mechanism that generates an interference with respect to (coherent) incident light such as an electromagnetic wave with an even wave surface, and measure a change in the incident light wave surface (phase) that is caused by a phase difference equivalent to one over several to several tens of a wavelength by measuring a fringe of the interference. An interferometer of this type is suitable in measuring a slight unevenness of, for example, a surface of a lens.

Among the wave surface measurement methods using interferences, X-ray phase imaging using an electromagnetic wave having a wavelength of several tens of nanometers or less, that is, an X-ray, has received considerable attention in recent years. Unlike a conventional X-ray absorption image created by imaging a contrast through absorption of an object, the X-ray phase imaging is a method for using an interference to detect a change in the phase of incident light which occurs when an X-ray penetrates an object. Such detection of a phase change enables the observation of a part of the inside of an object, such as a biological soft tissue, which has a low absorption coefficient, the observation being unachievable through the use of conventional absorption images.

X-ray Talbot interferometry (WO 2010/050483) is now described as an example of the X-ray phase imaging method. In an X-ray Talbot interferometer, an X-ray from a light source penetrates an object, which results in a change in the phase of incident light. The light penetrating the object is diffracted by a grating with a periodic pattern called "diffraction grating," forming a first interference pattern at a position distant from the diffraction grating by a predetermined distance, "Talbot length." A change in the incident light wave surface such as the one mentioned above is measured through comparative analysis of a change in the first interference pattern with and without the object.

The period of the pattern of such diffraction grating changes under the conditions including the apparatus length and the wavelength of the incident light. In case of a typical X-ray, the period of the pattern is in the order of several $\mu$m. The resultant first interference pattern also has a period in the order of several $\mu$m. In this case, a typical detector is not capable of detecting the first interference pattern because the resolution of the detector is several tens of $\mu$m at most. Therefore, a shield grating (absorption grating) with substantially the same pattern period as that of the first interference pattern is placed at a position where an interference pattern is formed. As a result of shielding a part of the first interference pattern with the shield grating, a second interference pattern with a pattern period of several hundreds of $\mu$m, i.e., a moire, is created. Changes in the interference pattern can be measured indirectly by detecting such moire with a detector.

There exist several phase retrieval methods such as a method for decoding a differential phase from a moire. One of these methods is a Fourier transform method. This method Fourier-transforms a moire image and acquires phase information from data in the spectrum matching the carrier frequency of the Fourier transform.

As described above, the Talbot interferometer needs to perform a phase retrieval method for acquiring and analyzing a moire image. In some cases, however, moire intensity information cannot be detected due to a defect in the detector or grating. In such a case, the phase information on the pixels located in the relevant sections will be lost. In addition, when a phase retrieval method such as a Fourier transform method for practically extracting information from a plurality of pixels on one or more images is used, there is a possibility that information on defective pixels not only expand in the defective pixel areas but also reach a neighborhood normal area.

Various methods have been proposed as a method for compensating the data of a defective pixel area. Japanese Patent Application Publication No. 2011-19591 discloses a method for compensating a defective pixel generated in a detector with the information on a neighborhood normal pixel. The method disclosed in Japanese Patent Application Publication No. 2011-19591 is effective for an apparatus that captures an absorption image, but is usually inadequate in compensating data using a type of apparatus that images a spatially periodic pattern, such as a Talbot interferometer. This is because, in case of an image with a periodic intensity pattern such as a moire, simply replacing the information on a defective pixel with the information on a neighborhood pixel is not enough to reproduce the period, and it is difficult to prevent the occurrence of the phenomenon described above in which the information on a defective pixel extends to a normal pixel area.

On the other hand, in case of a Talbot interferometer for directly analyzing a differential phase, an integration operation needs to be performed on a differential phase in order to calculate a phase. The wrong information generated due to the foregoing defect is amplified at the time of integration, causing qualitative, quantitative errors in the entire phase image.

## SUMMARY OF THE INVENTION

The present invention in its first aspect provides an image processing apparatus as specified in claims 1 to 11.

The present invention in its second aspect provides an imaging system as specified in claim 12.

The present invention in its third aspect provides a control method of an image processing apparatus as specified in claim 13.

The present invention in its fourth aspect provides a non-transitory computer readable storage medium storing a program as specified in claim 14.

The present invention can compensate a defective pixel contained in an image having a periodic pattern.

Further features of the present invention will become apparent from the following description of exemplary embodiments with reference to the attached drawings.

## BRIEF DESCRIPTION OF THE DRAWINGS

FIG. 1 is a diagram showing a configuration of an imaging system according to an embodiment;

FIG. 2A shows an example of an object;

FIG. 2B shows an example of a moire image;

FIG. 2C shows an example of a defective part within the moire image;

FIGS. 3A to 3D each show an example of phase retrieval according to Comparative Example 1;

FIGS. 4A to 4C each show an example of phase retrieval according to Comparative Example 2;

FIG. 5 is a block diagram showing a function associated with a defective pixel compensation process;

FIG. 6A is a flowchart of the defective pixel compensation process according to Example 1;

FIG. 6B is a flowchart of the defective pixel compensation process according to Example 2;

FIG. 7A shows an example of a Fourier pattern according to Example 1;

FIG. 7B shows an example of a filter;

FIG. 7C shows an example of a compensated moire image;

FIGS. 8A to 8C each show an example of phase retrieval according to Example 1; and

FIGS. 9A to 9C each show an example of phase retrieval according to Example 2.

## DESCRIPTION OF THE EMBODIMENTS

Embodiments of the present invention are contrived in view of the foregoing circumstances, and provide a technique for correcting an image containing a periodic pattern including a defective pixel by compensating the defective pixel.

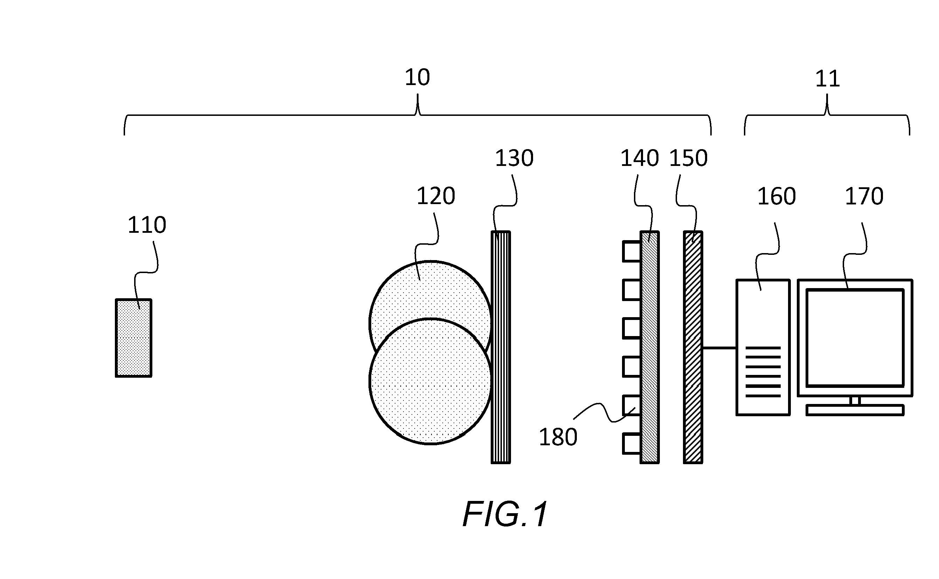

Embodiments of the present invention are described hereinafter. FIG. 1 is a diagram showing a configuration of an imaging system for Talbot X-ray phase imaging. The imaging system is configured by an imaging apparatus 10 configured by an X-ray Talbot interferometer, and an image processing apparatus 11 for processing an X-ray image acquired by the imaging apparatus 10. The imaging apparatus 10 has an X-ray source (light source) 110 for generating an X-ray, a diffraction grating 130 for diffracting the X-ray, a shield grating 140 for shielding a part of the X-ray, and a detector 150 for detecting the X-ray. The image processing apparatus 11 has an processing unit (computer) 160 connected to the imaging apparatus 10, and an image display apparatus 170 for displaying an image based on an processing result obtained by the processing unit 160.

The processing unit 160 can be configured by a general purpose computer equipped with hardware resources such as a central processing unit (CPU), a random access memory (RAM), and an auxiliary storage unit. Image processes, various computations and control, described hereinafter, are realized by causing the CPU to read and execute programs stored in the auxiliary storage unit. Note that all or part of the functions of the processing unit 160 can be configured by a circuit such as an application specific integrated circuit (ASIC).

An X-ray from the X-ray source 110 is diffracted by the diffraction grating 130, thereby forming interference patterns 180, in which the bright sections and the dark sections are arranged in an array direction, at a predetermined distance called "Talbot length".

A first interference pattern 180 formed by the diffraction grating 130 normally has a frequency of several $\mu$m to several tens of $\mu$m. The shield grating 140 having a frequency equal to or slightly different from that of the first interference pattern 180 is placed where the first interference pattern 180 is formed. Consequently, a moire is formed by the first interference pattern 180 and the shield grating 140, and as a result the period of the interference patterns can be expanded by several tens of μm or infinitely. The period of the moire can be determined appropriately in view of the phase retrieval method used and the pixel size of the detector. In the present embodiment, it is preferred that the period of the moire be twice or more of the pixel size and equal to or less than an imaging range of the detector. This moire (a pattern of spatial periodicity) is formed into a two-dimensional image by the detector 150, a two-dimensional image sensor. Such a mechanism enables imaging of an interference pattern having a period of several μm to several tens of μm by means of the detector 150 having a resolution of approximately several tens of square μm. However, when the detector 150 has a sufficiently high spatial resolution, the shield grating 140 may be omitted, and the first interference pattern 180 may be imaged directly.

Upon a measurement, an object 120 is placed in front of the diffraction grating 130. Due to its high transmissivity, an X-ray typically passes through the object 120, causing a phase change corresponding to the molecular structure and density of the substances through which the X-ray passes. The phase change has an impact on the arrangement of the first interference pattern 180, causing distortion in the moire with the shield grating 140. This distortion can be calculated by the processing unit 160 and acquired as phase differential information.

A method for acquiring a differential phase from a moire is now described. As this method for acquiring a differential phase image in a Talbot interferometer, there are various methods such as a phase shift method, a Fourier transform phase retrieval method, and so on, and the phase retrieval method is not particularly limited thereto. The present embodiment describes an example of using a two-dimensional grating with which differential phase image in two directions, an x-direction and a y-direction, can be acquired at once from a single moire. Other methods can be considered in which two imaging operations can be performed using a one-dimensional grating to acquire an x-direction differential phase image and a y-direction differential phase image separately. The following describes results that are obtained based on a Fourier transform phase retrieval method used as the phase retrieval method using a two-dimensional grating. The Fourier transform phase retrieval method is described in detail in WO 2010/050483.

An integration computation is executed using the acquired x-direction and y-direction differential phase images. An integration computation method conceivably includes a method for simply accumulating differential phase values from one end of a differential phase image, a method using Fourier transform, and the like. The present embodiment describes a Fourier integration method that takes advantage of the characteristics of a moire using a two-dimensional grating. Fourier integration method and Fourier transform phase retrieval method for obtaining the foregoing differential phases are in common in that they use Fourier transform, but are separate individual methods. For example, a phase shift method and a Fourier integration method may be used in combination.

The x-direction and y-direction differential phase images acquired by the foregoing method are represented as $D_x(x, y)$ and $D_y(x, y)$. The letters x and y represent the coordinates. The Fourier integration method uses a function such as Expression 1 shown below in which these two direction components are expressed as complex numbers.

$$D(x, y) = D_x(x, y) + iD_y(x, y) \qquad \text{(Expression 1)}$$

In this equation, i is the imaginary unit. Through computation of Expression 2 using this D, an integration value P with $D_x$ and $D_y$ as differential components is acquired.

$$P = \mathrm{F}^{-1}\left[\frac{\mathrm{F}[D(x, y)]}{k_x + ik_y}\right] \qquad \text{(Expression 2)}$$

In this equation, F[●] is an operator indicating that the function in the brackets is subjected to Fourier transform, $\mathrm{F}^{-1}$[●] an operator indicating that the function in the brackets is subjected to inverse Fourier transform, and $k_x$ and $k_y$ wavenumbers of a Fourier space. This Fourier transform method can reduce the impacts of noise and obtain an integration value of high precision.

The above is the summary of the Talbot X-ray phase imaging apparatus. The details and effects of the processes of the apparatus are described hereinafter in detail by using the simulation results obtained by the computer.

(Comparative Example 1)

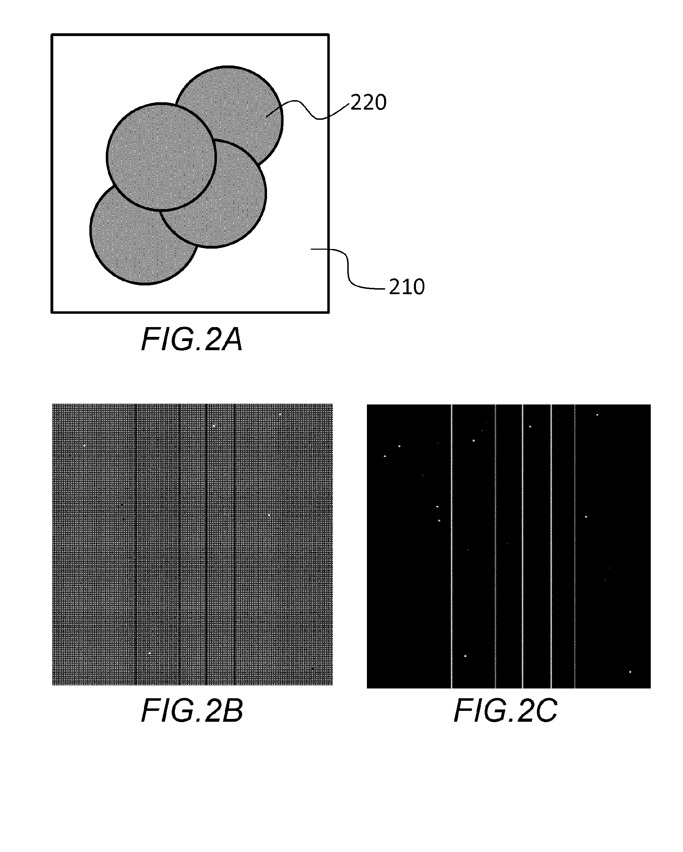

An example is simulated in which, as shown in FIG. 2A, a region 210 has an object 220 having four spheres overlapped on one another. FIG. 2B shows an example of a moire image obtained from the object 220 shown in FIG. 2A. This example assumes that the moire image has constant defective pixels. FIG. 2C shows a map of the defective pixels, in which the white parts are the defective pixels. In this simulation, the defective pixels are each set as a pixel for which an X-ray detection intensity is higher than a predetermined value TH1 (hot spot) or a pixel for which the X-ray detection intensity is lower than a predetermined value TH2 (< TH1) (dark spot). In other words, in the moire image, the pixels having pixel values within the range of TH1 to TH2 are considered as normal pixels, whereas the pixels having pixel values outside this range are considered as defective pixels. However, it should be noted that a method for detecting defective pixels is not the essence of the present invention; thus, various methods for acquiring defective pixels can be used. Moreover, the defective pixels indicate not only abnormalities of the detector but also pixels in the regions that are generated due to the abnormalities or configurations of the grating and the like and cannot be use as data.

Let it assume that roughly three types of defects are generated as the defective pixels: an independent point defect surrounded by the normal pixels; a surface defect in which point defects are present continuously on a plane (suppose that the size of the surface defects is 3 × 3); and a line defect in which point defective pixels are arranged in a line.

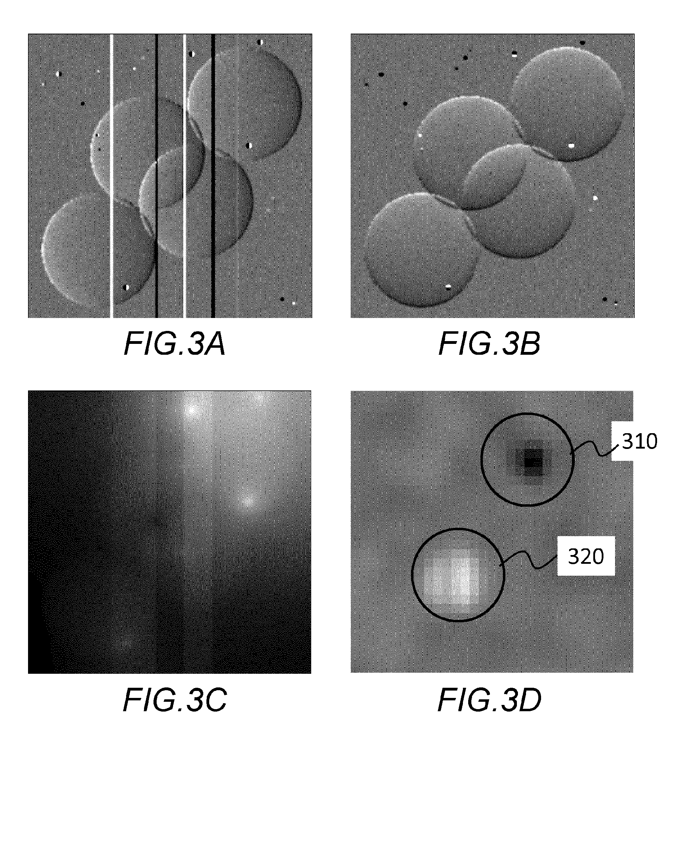

Comparative Example 1 introduces an example in which phase retrieval is performed without executing any types of defect compensation. FIGS. 3A to 3D each show a result of Comparative Example 1. FIGS. 3A and 3B respectively show an x-axis direction differential phase image and a y-axis direction differential phase image, which are obtained by performing phase retrieval without executing defect compensation. FIG. 3C shows an integral image acquired using the Fourier integration method. As shown in FIGS. 3A, 3B and 3C, the defective pixels of the moire image have impacts on the phase images and serious impacts on the integral image.

FIG. 3D is a diagram showing enlargements of the parts of the phase image of FIG. 3A that correspond to the point defects. In the moire image, the point defects are each an independent 1 × 1 defective pixel, and an abnormality of the numerical value thereof extends to the outside of defective pixels (regions 310 and 320). This phenomenon shows that the defective pixel areas expand on the phase-recovered image. In other words, this phenomenon indicates that it is inefficient to perform the method for compensating the defective pixels after executing phase retrieval.

(Comparative Example 2)

Comparative Example 2 introduces an example of performing compensation and phase retrieval on a defective pixel portion of the input image by means of a method for compensating it with a neighborhood pixel, such as the method described in Japanese Patent Application Publication No. 2011-19591. In this example, a 5 × 5 median filter is used to calculate a representative value of the defective pixel portion. First of all, the moire image (FIG. 2B), the original image, is median-filtered to create data. Subsequently, the defective pixels in the original image are compensated by being replaced with the pixels of the median-filtered data that are located in the positions corresponding to the positions of the defective pixels, and then phase retrieval is executed on the resultant pixels.

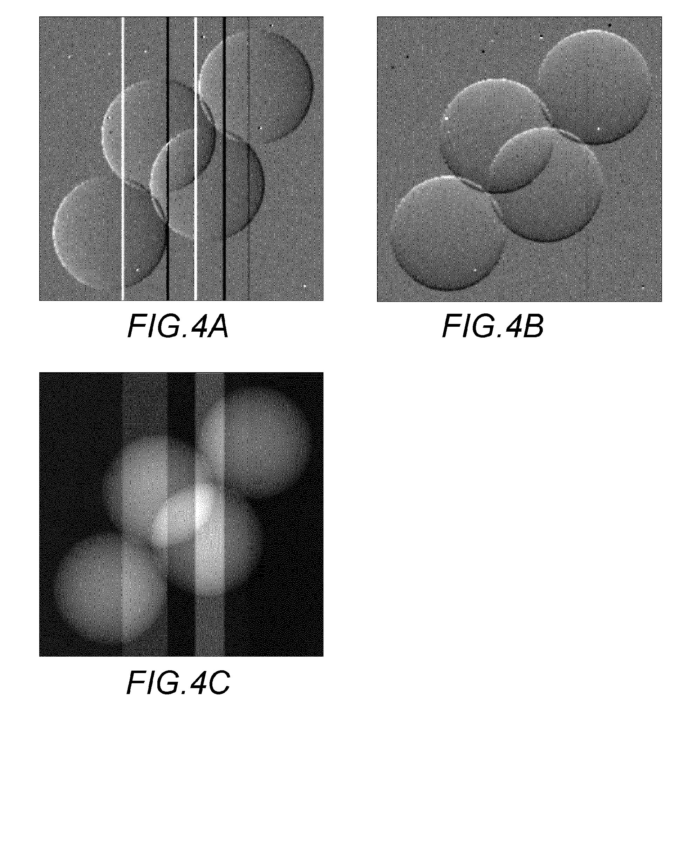

FIGS. 4A to 4C show the results of Comparative Example 2. FIGS. 4A and 4B show, respectively, an x-axis direction differential phase image and a y-axis direction differential phase image which are obtained as a result of phase retrieval by the method of Comparative Example 2. FIG. 4C is an integral image acquired using the Fourier integration method. Although the impacts of the defective pixel portions are lighter as compared to Comparative Example 1, the impacts of the defects still reach the normal pixel areas in the integral image. The line defects in particular generate uneven stripes in the integral image.

(Example 1)

There is a possibility that the method of compensation using the information on a neighborhood pixel as described in Comparative Example 2 is not suitable for a Talbot interferometer. The reason lies in the fact that a Talbot interferometer uses an interference fringe (a pattern of spatial periodicity) called moire. The reason of this is because, since the intensity of the moire periodically changes location by location, the defective parts need to be compensated by being replaced with highly probable values by using the values of the interference fringes, in view of such periodical changes. The compensation method according to the present example has a process of compensating the defective parts of the moire image in view of the moire periodicity, to reproduce the moire, thereby reducing the impacts of the defective pixel portions more compared to Comparative Examples 1 and 2.

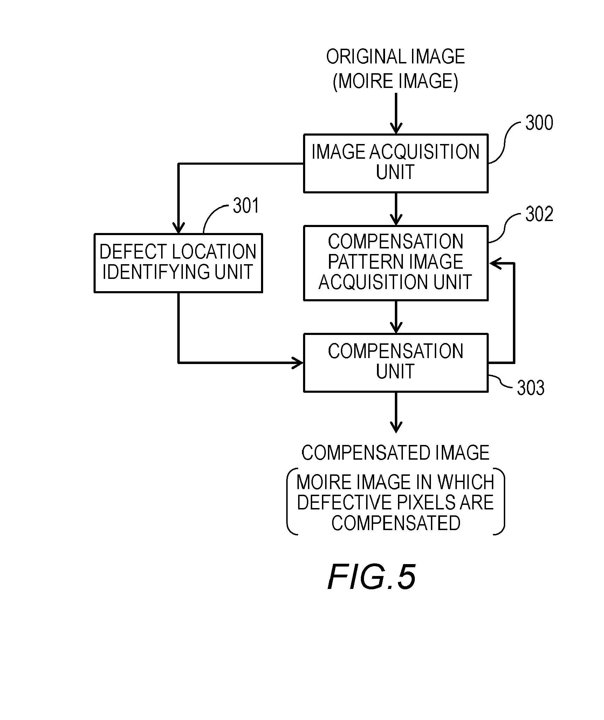

FIG. 5 is a block diagram showing a function associated with a defective pixel compensation process (correction process) of the image processing apparatus 11. The function is configured by an image acquisition unit 300, a defect location identifying unit 301, a compensation pattern image acquisition unit 302, and a compensation unit 303. The image acquisition unit 300 functions to acquire the original image (moire image) that includes a periodic pattern including a defective pixel. The defect location identifying unit 301 functions to identify the position of a defective pixel of the original image. The compensation pattern image acquisition unit 302 functions to acquire a compensation pattern image. The compensation pattern image is an image having the same periodic pattern as the original image, in which the impact of a defective pixel thereof is lower (or no defective pixels) than that of the original image. The compensation unit 303 functions to generate a compensated image in which the defective pixel is compensated, by using the original image and the compensation pattern image.

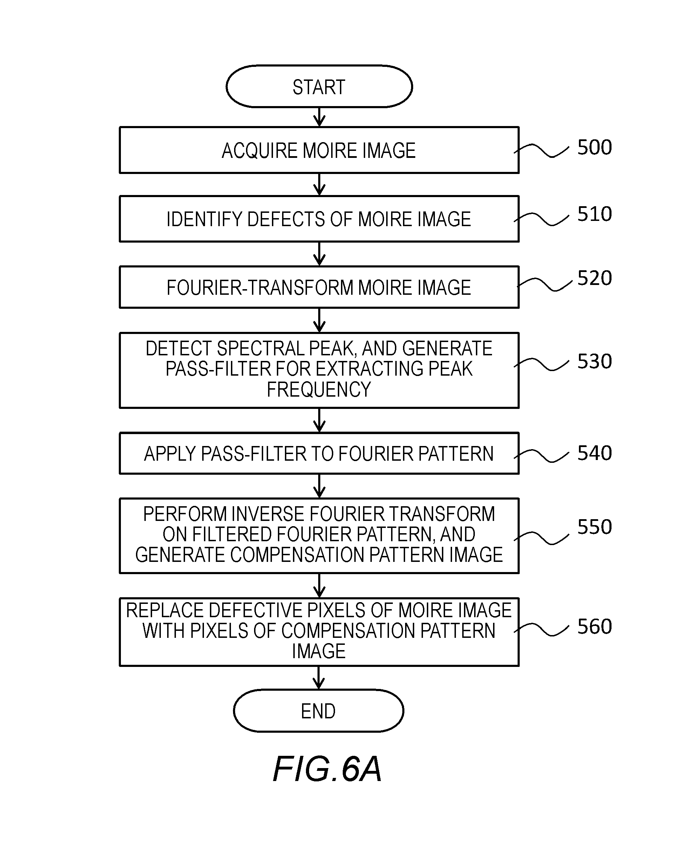

In Example 1, the compensation pattern image is generated by extracting, from the original image, a spatial frequency component corresponding to the moire, and then the defective pixels of the original image are replaced with the pixels of the compensation pattern image that are located in the positions corresponding to the positions of the defective pixels of the original image, thereby compensating the defective pixels. These functions are described hereinafter in detail with reference to the flowchart shown in FIG. 6A.

First, the image acquisition unit 300 acquires the moire image (original image) from the imaging apparatus 10 (step 500). Subsequently, the defect location identifying unit 301 determines defective parts of the input moire image (510). Specifically, as described in Comparative Example 1, the defect location identifying unit 301 considers the pixel of the moire image that have the pixels values of TH1 to TH2 as the normal pixels, and determines the pixels outside this pixel value range as the defective pixels. However, not only this method but also any methods may be used in order to identify defective parts. For instance, since the positions of defective pixels depend on the imaging apparatus 10, the information on the positions of the defective pixels of the imaging apparatus 10 may be stored beforehand in the storage unit, and then in step 510 the information on the positions may be read from the storage unit.

Next, the compensation pattern image acquisition unit 302 performs Fourier transform on the moire image (step 520). As a result of Fourier transform, a Fourier pattern (also referred to as "Fourier image," "power spectrum," or "spatial-frequency image") of the moire is obtained. In the Fourier pattern, peaks appear in frequency components corresponding to the moire period. The positions of the peaks and the number of peaks vary depending on the type of the moire. The compensation pattern image acquisition unit 302 detects spectral peaks from the Fourier pattern, to generate band-pass filters, the centers of which match the respective peak positions (peak frequencies) (step 530). The compensation pattern image acquisition unit 302 then filters the Fourier pattern with a combination of these band-pass filters and thereby extracts the information on the portion of the peak frequencies, i.e., the spatial frequency components corresponding to the moire (step 540). This filtering operation is the same as the operation for eliminating or reducing the spatial frequency components of the defective pixels, which are the image information other than the moire pattern included in the original image.

Although the accuracy of the moire pattern can be improved by increasing the widths of the pass filters, there is a possibility that excessively wide pass filters are affected by the defective parts. Therefore, the widths (sizes) of the filters need to be set at appropriate values. For example, Gaussian filters defined by Expression 3 are used as the pass filters, in which the value of a variance $\sigma$ representing the width (radius) of each pass filter is set at 1 (pixel unit).

$$F = \exp\left[-\frac{x^2 + y^2}{2\sigma^2}\right] \qquad \text{(Expression 3)}$$

Next, the compensation pattern image acquisition unit 302 performs inverse Fourier transform on the filtered Fourier pattern (step 550), thereby generating a compensation pattern image in which the defective areas of the original image are replaced with highly probable data. Finally, the compensation unit 303 compensates the defective pixels by replacing the data of the defective pixels of the original moire image with the data of the pixels of the compensation pattern image obtained in step 550 which are located in the positions corresponding to the positions of the defective pixels (step 560).

With the foregoing process, a compensated image in which the defective pixels are compensated (also referred to as "compensated moire image," hereinafter) can be obtained. According to this method, moire information of the defective pixel areas of the original moire image are restored. Therefore, a natural image can be generated even when a phase retrieval operation is performed.

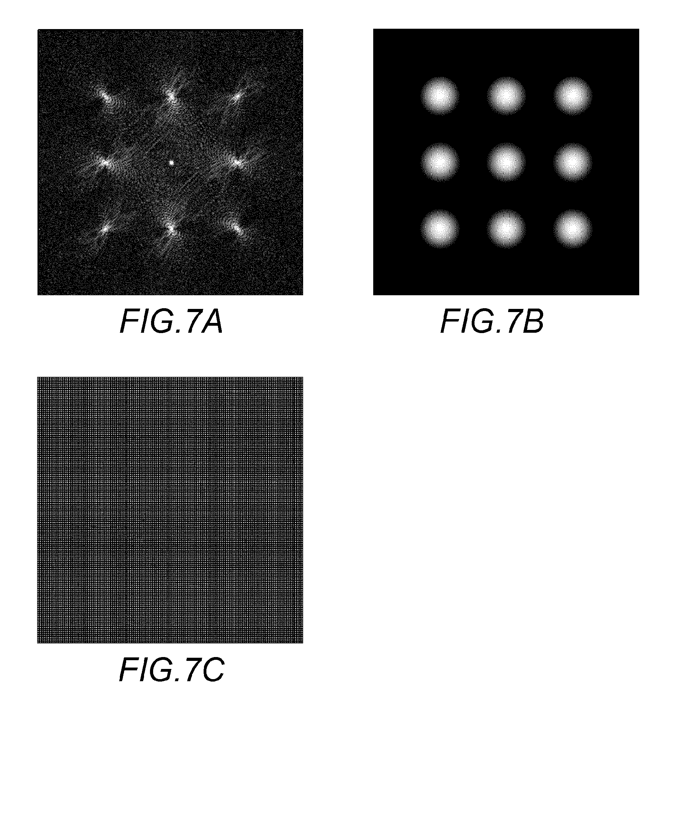

FIG. 7A shows a Fourier pattern that is obtained by Fourier-transforming the moire image shown in FIG. 2B. To be exact, the diagram shows the absolute value of the Fourier pattern (specifically, the absolute value is drawn in logarithm scale in order to enhance the visibility of the diagram), since the value obtained after Fourier transform is typically a complex number. In this case, a total of nine peaks appear at the positions corresponding to the moire period. FIG. 7B shows an example of a filer obtained by combining Gaussian pass filters corresponding to the nine peaks respectively. A deviation $\sigma$ representing the radius of each of the Gaussian pass filters is 1 pixel (note that the widths of the pass filters seem wider than the actual widths because the pass filters are drawn in logarithm scale in order to enhance the visibility of the diagram). The Fourier pattern obtained by overlapping the filters is subjected to inverse Fourier transform to generate a compensation pattern image. FIG. 7C shows a compensated moire image obtained by replacing the defective pixels of the moire image shown in FIG. 2B with the pixels of the compensation pattern image which are located at the positions corresponding to the positions of the defective pixels.

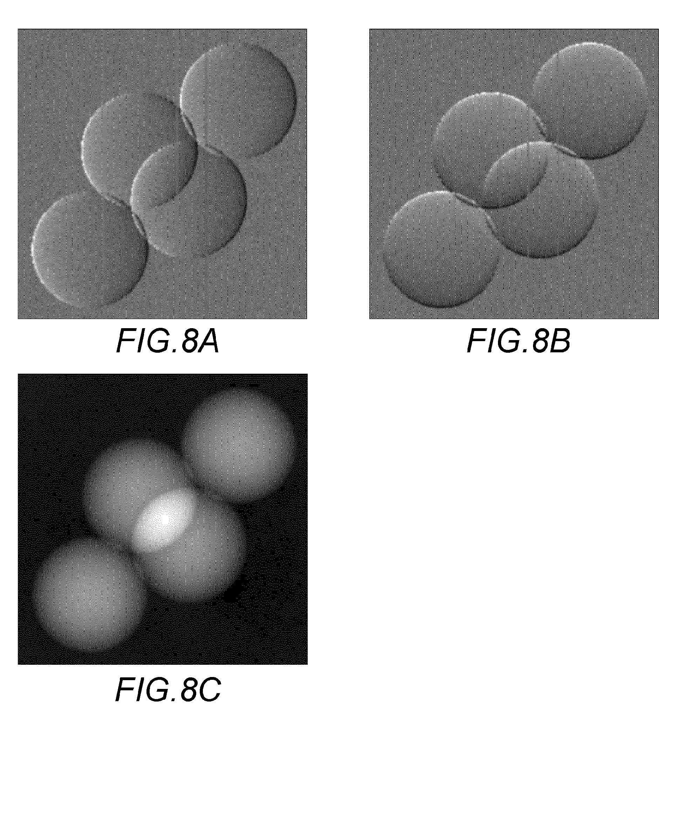

FIGS. 8A and 8B show an x-axis direction differential phase image and a y-axis direction differential phase image, respectively, which are subjected to phase retrieval using the compensated moire image shown in FIG. 7C. FIG. 8C shows an integral image acquired using the Fourier integration method. It is clear that the distortion of the differential phase images and the distortion of the integrated phase image illustrated in Comparative Examples 1 and 2 are reduced, proving the effects of the method described in Example 1.

(Example 2)

Example 2, proposes a method for repeating the method illustrated in Example 1 a certain number of times. In other words, a process of generating a new compensation pattern image from the compensated moire image and compensating the defective pixels of the original moire image by means of the new compensation pattern image is executed repeatedly. This process can improve the correction effects (compensation effects) and achieve the effect of correcting line defects in particular, this correction effect being better than that of Example 1.

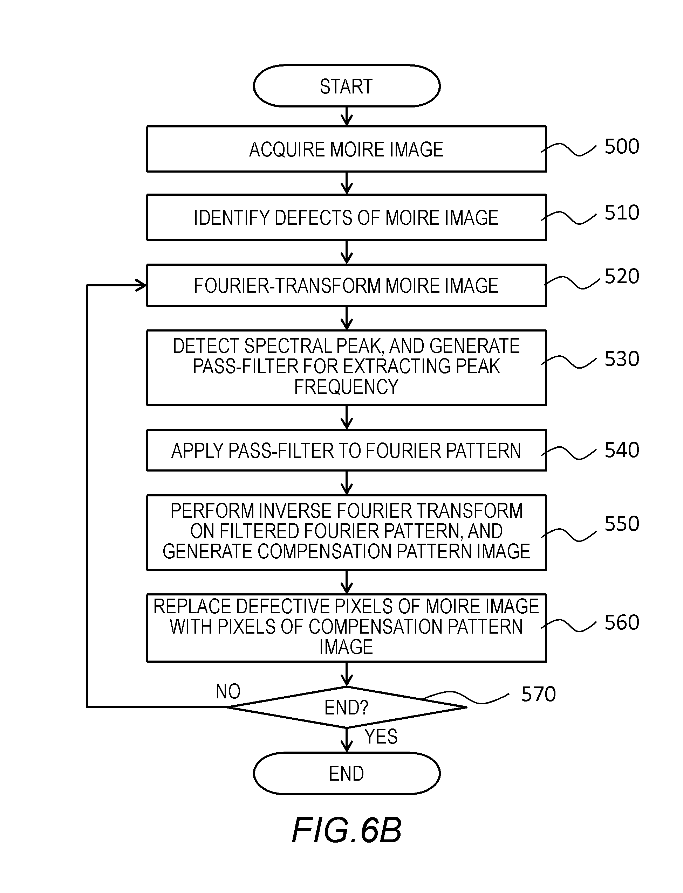

FIG. 6B shows a flowchart of the process of Example 2. The difference with the process of Example 1 (FIG. 6A) is that in Example 2, steps 520 to 560 are repeated under a certain condition (step 570). Note that, for the second subsequent times, step 520 uses the compensated moire image obtained in step 560. As a result, the ability to reproduce the moire pattern of the compensation pattern image can be improved.

The repetition in step 570 may be ended in any condition. In Example 2, the number of times to repeat the steps is set before hand (e.g., 20 time), and the process is ended once the steps are repeated the set number of times. In another method, some sort of an evaluation formula or evaluation value may be set beforehand to determine when to conclude the process. For instance, a total of differences among the pixels of the previous compensated moire image and the pixels of the current compensated moire image may be taken as an evaluated value, and when this evaluated value becomes equal to or lower than a predetermined threshold (i.e., when the effect of improving the efficiency of compensation through the repeating process diminishes), the repeating process may be ended.

Furthermore, in Example 2, the width of each of Gaussian pass filters in step 530 (the filter radius) is increased gradually each time the process is repeated (specifically, the value of the Gaussian variance $\sigma$ is increased to 20 by one). Increasing the width of each filter enables extraction of more of the information on the object superimposed on the moire pattern, improving the ability to reproduce both the moire pattern and the object information of the compensation pattern image. Note that, in the first half of the repeating process, the narrow width of each filter inhibits extraction of the information on the defective pixels as much as possible, and the width of each filter is increased as the correction of the defective pixels progresses, to give priority to extraction of the information on the object. Therefore, a more reasonable compensation pattern image can be obtained, improving the accuracy of the compensated moire image.

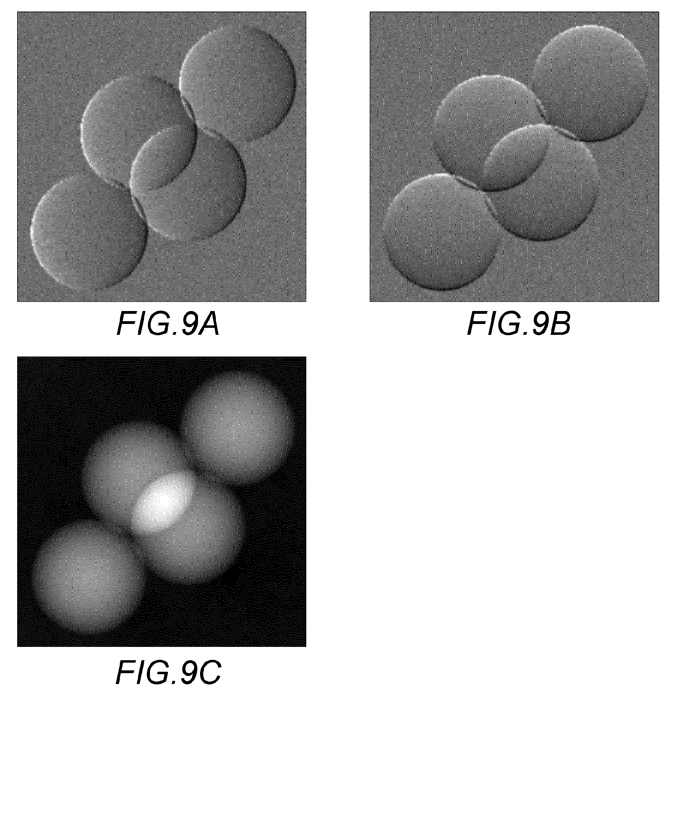

FIGS. 9A and 9B show respectively an x-axis direction differential phase image and a y-axis direction differential phase image which are obtained as a result of phase retrieval executed using the compensated moire image obtained eventually by the method of the present example. FIG. 9C shows an integral image acquired using the Fourier integration method. As shown in the diagram, the distortion (line-shaped errors) is alleviated more as compared to the result obtained in Example 1, indicating that the effect is enhanced more than in Example 1.

The effects of these examples are shown quantitatively in Table 1. Table 1 shows the results that are obtained by comparing the differential phase images and integral images of the four examples, Comparative Examples 1 and 2 and Examples 1 and 2, with the values of the same assuming that there were no defective pixels. The values shown in Table 1 are each the mean square of the difference between the pixel value of each image and the pixel value assuming that there are no defective pixels, and the lower the values the better the results.

[Table 1]

|  | Difference in X Differential Phase Image | Difference in Y Differential Phase Image | Difference in Integral Image |
| --- | --- | --- | --- |
| Comparative Example 1 | 0.0611 | 0.0067 | 22.0559 |
| Comparative Example 2 | 0.0344 | 0.0037 | 1.3143 |
| Example 1 | 0.0032 | 0.0031 | 0.4149 |
| Example 2 | 0.0026 | 0.0027 | 0.4086 |

It is clear that the methods of Examples 1 and 2 can compensate the defective pixels at higher quality than Comparative Examples 1 and 2.

(Other Examples)

The foregoing examples are merely the specific examples of the present invention and therefore are not intended to limit the scope of the present invention to these specific examples.

Any images can be used as the compensation pattern image as long as they contain the same periodic patterns as those of an original image, and various configurations are conceivable as the method, means, timing (time) and the like for acquiring the compensation pattern image. For example, the foregoing examples illustrate the method of generating a compensation pattern image from an original image, and this method has the advantage that the information on the object on the original image can be reproduced in the compensation pattern image. However, the compensation pattern image may also be generated from another image obtained by the same imaging apparatus (e.g., an image captured without an object, an image captured using a phantom (model), or the like). Alternatively, a moire pattern that is calculated logically from the grating configuring the imaging apparatus or the parameter of the detector can be used as the compensation pattern image. In such a case where the compensation pattern image is generated from an image other than the original image, the compensation pattern image that is generated beforehand may be stored in the storage unit. In this case, in place of steps 520 to 550 described in Example 1 (FIG. 6A), only the process of reading the relevant compensation pattern image from the storage unit may be executed, reducing the processing time. Note that the present invention and the present specification consider this process of reading the previously generated compensation pattern image as the process of acquiring a compensation pattern image.

The foregoing examples use Gaussian pass filters, but the shapes of the filters are not limited. Specifically, a filter consisting of the Sinc function or simple cylindrical window function may be used. In addition, the foregoing examples detect the peaks in the Fourier pattern and set the filters in accordance of the peak frequencies. This is because, since the peak frequencies can fluctuate depending on drift of the imaging apparatus or the object, setting the positions of the filters in accordance with the spectral peaks of the original image as described in the foregoing examples brings about the advantage of being able to generate a more accurate compensation pattern image. However, when the fluctuations of the peak frequencies can be ignored, a filter with its position fixed may be used. In that case, step 530 can be omitted, reducing the processing time.

The foregoing examples have illustrated an X-ray Talbot interferometer using a two-dimensional grating; however, the applicable scope of the present invention is not limited thereto. The present invention can be applied to, for example, interferometers using one-dimensional gratings and interferometers using various types of electromagnetic waves having various wavelengths other than X-rays (e.g., optical interferometers). In addition to the interferometers, the defective pixel compensation method of the present invention can suitably be applied to an image that is obtained by imaging a spatially periodic pattern formed by an electromagnetic wave reflected from or penetrating an object. An imaging apparatus to which the present invention can be applied may be configured separately from the X-ray source or electromagnetic wave source and may be configured to be able to perform imaging in combination with the X-ray source or electromagnetic wave source. In the present invention and the present specification, an imaging apparatus may be any apparatus capable of imaging a periodic pattern and therefore is not limited to an apparatus that forms the information of an object into an image.

The image processing apparatus described above can specifically be implemented by software (a program) or hardware. For example, a computer program may be stored in the computer (microcomputer, CPU, MPU, FPGA, etc.) of the image processing apparatus, and each process may be realized by causing the computer to execute the computer program. In addition, it is preferred that a special purpose process such as an ASIC may be provided in order to cause a logical circuit to realize all or part of the processes according to the present invention. The present invention can be applied to a server in a cloud environment.

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

An image processing apparatus includes: an image acquisition unit configured to acquire an original image containing a periodic pattern including a defective pixel; a compensation pattern image acquisition unit configured to acquire a compensation pattern image containing a periodic pattern same as that of the original image; a defect location identifying unit configured to identify a position of the defective pixel in the original image; and a compensation unit configured to compensate the identified defective pixel of the original image with a pixel on the compensation pattern image that is located in a position corresponding to the position of the defective pixel, to generate a compensated image in which the compensated defective pixel is compensated.

**Claims**

1. An image processing apparatus, comprising:

    an image acquisition unit configured to acquire an original image containing a periodic pattern including a defective pixel;
    a compensation pattern image acquisition unit configured to acquire a compensation pattern image containing a periodic pattern same as that of the original image;
    a defect location identifying unit configured to identify a position of the defective pixel in the original image; and
    a compensation unit configured to compensate the identified defective pixel of the original image with a pixel on the compensation pattern image that is located in a position corresponding to the position of the defective pixel, to generate a compensated image in which the compensated defective pixel is compensated.

2. The image processing apparatus according to claim 1, wherein
the compensation pattern image acquisition unit generates the compensation pattern image by extracting a spatial-frequency component corresponding to the periodic pattern from the original image.

3. The image processing apparatus according to claim 2, wherein
the compensation pattern image acquisition unit Fourier-transforms the original image to obtain a Fourier pattern, extracts information on a portion of a peak frequency by applying a band-pass filter to the Fourier pattern, and generates the compensation pattern image by performing inverse Fourier transform on the extracted information.

4. The image processing apparatus according to claim 3, wherein the compensation pattern image acquisition unit detects a peak frequency from the Fourier pattern and sets the band-pass filter around the detected peak frequency.

5. The image processing apparatus according to claim 3 or 4, wherein a process, in which the compensation pattern image acquisition unit generates a new compensation pattern image from the compensated image and the compensation unit generates a new compensated image by compensating the defective pixel of the original image using the new compensation pattern image, is repeated.

6. The image processing apparatus according to claim 5, wherein the compensation pattern image acquisition unit increases a width of the band-pass filter each time the process is repeated.

7. The image processing apparatus according to claim 1, wherein the compensation pattern image acquisition unit reads, from a storage unit that stores compensation pattern images generated beforehand, a compensation pattern image containing a periodic pattern same as that of the original image.

8. The image processing apparatus according to any one of claims 1 to 7, wherein the defect location identifying unit identifies a pixel of the original image that has a pixel value outside a predetermined range, as a defective pixel.

9. The image processing apparatus according to any one of claims 1 to 8, wherein the original image is an image that is obtained by imaging a spatial periodic pattern formed by an electromagnetic wave reflected from or penetrating an object.

10. The image processing apparatus according to any one of claims 1 to 9, wherein the original image is an image that is obtained by an imaging apparatus that forms an interference pattern through interference of an electromagnetic wave reflected from or penetrating an object and images the interference pattern with a detector.

11. The image processing apparatus according to claim 10, wherein the imaging apparatus is an X-ray Talbot interferometer.

12. An imaging system, comprising:

an imaging apparatus configured to form an interference pattern through interference of an electromagnetic wave reflected from or penetrating an object, and to image the interference pattern with a detector; and
the image processing apparatus according to any one of claims 1 to 11.

13. A control method of an image processing apparatus, comprising the steps of:

acquiring an original image containing a periodic pattern including a defective pixel;
acquiring a compensation pattern image containing a periodic pattern same as that of the original image;
identifying a position of the defective pixel in the original image; and
compensating the identified defective pixel of the original image with a pixel on the compensation pattern image that is located in a position corresponding to the position of the defective pixel, to generate a compensated image in which the compensated defective pixel is compensated.

14. A non-transitory computer readable storage medium storing a program, which causes a computer to execute each of the steps of the control method of an image processing apparatus according to claim 13.

*FIG.1*

FIG.2A

FIG.2B

FIG.2C

*FIG.3A*

*FIG.3B*

*FIG.3C*

*FIG.3D*

FIG.4A

FIG.4B

FIG.4C

ORIGINAL IMAGE
(MOIRE IMAGE)

↓

IMAGE ACQUISITION
UNIT — 300

↓

DEFECT LOCATION
IDENTIFYING UNIT — 301

COMPENSATION
PATTERN IMAGE
ACQUISITION UNIT — 302

↓

COMPENSATION
UNIT — 303

↓

COMPENSATED IMAGE

⎡ MOIRE IMAGE IN WHICH ⎤
⎢ DEFECTIVE PIXELS ARE ⎥
⎣ COMPENSATED ⎦

*FIG.5*

```
                    ┌─────────────┐
                    │    START    │
                    └──────┬──────┘
                           ↓
        ┌──────────────────────────────────────┐
        │        ACQUIRE MOIRE IMAGE            │ ── 500
        └──────────────────┬───────────────────┘
                           ↓
        ┌──────────────────────────────────────┐
        │     IDENTIFY DEFECTS OF MOIRE IMAGE   │ ── 510
        └──────────────────┬───────────────────┘
                           ↓
        ┌──────────────────────────────────────┐
        │     FOURIER-TRANSFORM MOIRE IMAGE     │ ── 520
        └──────────────────┬───────────────────┘
                           ↓
        ┌──────────────────────────────────────┐
        │  DETECT SPECTRAL PEAK, AND GENERATE   │
        │  PASS-FILTER FOR EXTRACTING PEAK      │ ── 530
        │  FREQUENCY                            │
        └──────────────────┬───────────────────┘
                           ↓
        ┌──────────────────────────────────────┐
        │   APPLY PASS-FILTER TO FOURIER PATTERN│ ── 540
        └──────────────────┬───────────────────┘
                           ↓
        ┌──────────────────────────────────────┐
        │  PERFORM INVERSE FOURIER TRANSFORM    │
        │  ON FILTERED FOURIER PATTERN, AND     │ ── 550
        │  GENERATE COMPENSATION PATTERN IMAGE  │
        └──────────────────┬───────────────────┘
                           ↓
        ┌──────────────────────────────────────┐
        │ REPLACE DEFECTIVE PIXELS OF MOIRE IMAGE│
        │ WITH PIXELS OF COMPENSATION PATTERN   │ ── 560
        │ IMAGE                                 │
        └──────────────────┬───────────────────┘
                           ↓
                    ┌─────────────┐
                    │     END     │
                    └─────────────┘
```

*FIG.6A*

```
                        ┌──────────────┐
                        │    START     │
                        └──────┬───────┘
                               ↓
              ┌──────────────────────────────────┐
              │      ACQUIRE MOIRE IMAGE          │──── 500
              └──────────────┬───────────────────┘
                             ↓
              ┌──────────────────────────────────┐
              │   IDENTIFY DEFECTS OF MOIRE IMAGE │──── 510
              └──────────────┬───────────────────┘
                             ↓
     ┌───→   ┌──────────────────────────────────┐
     │       │   FOURIER-TRANSFORM MOIRE IMAGE   │──── 520
     │       └──────────────┬───────────────────┘
     │                      ↓
     │       ┌──────────────────────────────────┐
     │       │  DETECT SPECTRAL PEAK, AND GENERATE│
     │       │  PASS-FILTER FOR EXTRACTING PEAK   │──── 530
     │       │            FREQUENCY               │
     │       └──────────────┬───────────────────┘
     │                      ↓
     │       ┌──────────────────────────────────┐
     │       │  APPLY PASS-FILTER TO FOURIER PATTERN│──── 540
     │       └──────────────┬───────────────────┘
     │                      ↓
     │       ┌──────────────────────────────────┐
     │       │  PERFORM INVERSE FOURIER TRANSFORM│
     │       │  ON FILTERED FOURIER PATTERN, AND │──── 550
     │       │ GENERATE COMPENSATION PATTERN IMAGE│
     │       └──────────────┬───────────────────┘
     │                      ↓
     │       ┌──────────────────────────────────┐
     │       │ REPLACE DEFECTIVE PIXELS OF MOIRE IMAGE│
     │       │  WITH PIXELS OF COMPENSATION PATTERN │──── 560
     │       │            IMAGE                   │
     │       └──────────────┬───────────────────┘
     │        NO            ↓
     └──────────────◇  END? ◇──── 570
                        │
                        │ YES
                        ↓
                 ┌──────────────┐
                 │     END      │
                 └──────────────┘
```

*FIG.6B*

FIG.7A

FIG.7B

FIG.7C

FIG.8A

FIG.8B

FIG.8C

*FIG.9A*

*FIG.9B*

*FIG.9C*

Europäisches
Patentamt

European
Patent Office

Office européen
des brevets

# EUROPEAN SEARCH REPORT

Application Number

EP 15 16 1087

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/235775 A1 (TADA TAKUJI [JP]) 29 September 2011 (2011-09-29) | 1,8-14 | INV. H04N5/217 H04N5/32 A61B6/00 G06T7/00 H04N5/367 |
| A | * paragraph [0083] - paragraph [0098] * * figures 6-9 * | 2-7 | |
| X | US 2012/155610 A1 (MURAKOSHI DAI [JP] ET AL) 21 June 2012 (2012-06-21) | 1,8-14 | |
| A | * paragraph [0112] - paragraph [0113] * * paragraph [0160] - paragraph [0162] * * figure 2 * | 2-7 | |
| X | WO 2013/099467 A1 (FUJIFILM CORP [JP]) 4 July 2013 (2013-07-04) | 1,8-14 | |
| A | * paragraph [0025] * * paragraph [0068] - paragraph [0078] * * figures 6-9 * | 2-7 | |

TECHNICAL FIELDS
SEARCHED    (IPC)

H04N
A61B
G06T

The present search report has been drawn up for all claims

| Place of search | Date of completion of the search | Examiner |
|---|---|---|
| The Hague | 11 August 2015 | Potin, Delphine |

## ANNEX TO THE EUROPEAN SEARCH REPORT
## ON EUROPEAN PATENT APPLICATION NO.

EP 15 16 1087

This annex lists the patent family members relating to the patent documents cited in the above-mentioned European search report.
The members are as contained in the European Patent Office EDP file on
The European Patent Office is in no way liable for these particulars which are merely given for the purpose of information.

11-08-2015

| Patent document cited in search report | | Publication date | Patent family member(s) | | Publication date |
|---|---|---|---|---|---|
| US 2011235775 | A1 | 29-09-2011 | JP | 5378335 B2 | 25-12-2013 |
| | | | JP | 2011223545 A | 04-11-2011 |
| | | | US | 2011235775 A1 | 29-09-2011 |
| US 2012155610 | A1 | 21-06-2012 | CN | 102551769 A | 11-07-2012 |
| | | | JP | 2012143549 A | 02-08-2012 |
| | | | US | 2012155610 A1 | 21-06-2012 |
| WO 2013099467 | A1 | 04-07-2013 | JP | 2013150780 A | 08-08-2013 |
| | | | WO | 2013099467 A1 | 04-07-2013 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010050483 A

- JP 2011019591 A